# FASCICULE DE BREVET EUROPEEN

(11) **EP 1 248 567 B1**
(45) Date de publication et mention de la délivrance du brevet: **31.03.2004**
(21) Numéro de dépôt: 01907632.2
(22) Date de dépôt: 18.01.2001
(51) Int. Cl.: A61B 17/04

(54) **DISPOSITIF PERCUTANE POUR LE TRAITEMENT DE L'INCONTINENCE URINAIRE D'EFFORT DE LA FEMME PAR BANDELETTE SOUS URETRALE**
PERKUTANE VORRICHTUNG ZUR BEHANDLUNG VON STRESSBEDINGTER HARNINKONTINENZ BEI FRAUEN MIT SUBURETHRALEN STREIFEN
TRANSCUTANEOUS DEVICE FOR TREATING FEMALE URINARY INCONTINENCE BY SUB-URETHRAL STRIP

(30) Priorité: 21.01.2000 US 489336; 05.10.2000 FR 0012753
(43) Date de publication de la demande: 16.10.2002
(73) Titulaire: SOFRADIM PRODUCTION, 01600 Trévoux (FR)
(72) Inventeur: SCETBON, Victor, F-75017 Paris (FR)
(74) Mandataire: Maureau, Philippe
(86) Numéro de dépôt international: PCT/FR2001/000167
(87) Numéro de publication internationale: WO 2001/052750

(56) Documents cités:
- WO-A-99/62406
- US-A- 5 112 344
- US-A- 5 899 909

## Description

La présente invention se rapporte aux problèmes d'incontinence urinaire chez la femme et plus particulièrement aux problèmes d'incontinence urinaire d'effort. L'invention concerne plus particulièrement un dispositif percutané pour le traitement de l'incontinence urinaire d'effort de la femme par bandelette sous urétrale.

Ces problèmes sont actuellement traités au cours d'interventions chirurgicales sous anesthésie locale, régionale ou générale et consistent à implanter une bandelette de manière à réaliser un soutènement sans tension de l'urètre.

Grâce à l'anesthésie régionale ou locale, le chirurgien peut vérifier immédiatement la restauration de la continence avec la participation de la patiente.

Une telle intervention est effectuée avec un dispositif adéquat, comprenant des instruments spécialisés.

Il est notamment connu d'utiliser pour le traitement de l'incontinence urinaire, une bandelette susceptible d'être implantée sous le canal urétral, et une gaine entourant la bandelette, ladite gaine étant retirée de ladite bandelette après l'implantation de cette dernière.

On connaît un dispositif pour le traitement de l'incontinence urinaire d'effort chez la femme, comprenant :
- un moyen souple et allongé de soutien de l'urètre, comportant une bandelette et une gaine de protection enveloppant à plat ladite bandelette,
- et une aiguille perforante présentant une extrémité distale active et une extrémité proximale liée à une première extrémité du moyen souple.

On connaît ainsi, par l'intermédiaire du document US-A-5 899 909, une méthode de traitement ainsi qu'un dispositif de traitement de l'incontinence. Le dispositif décrit, qui permet de poser une bandelette sous l'urètre, comporte deux aiguilles spéciales. Ces dernières sont successivement montées, par vissage, sur un introducteur réutilisable en acier, composé d'une poignée et d'une tige filetée de manipulation, laquelle permet de manipuler successivement chacune desdites aiguilles. Chaque aiguille est fixée à l'une des extrémités de l'ensemble bandelette-gaine.

Chaque extrémité de l'ensemble bandelette-gaine est fixée sur une partie tronconique d'une extrémité de l'aiguille correspondante, à l'aide d'un anneau en polymère rétracté ou collé.

L'implantation de la bandelette est donc effectuée en introduisant chacune des aiguilles à partir d'une courte incision de la paroi vaginale antérieure, et ce de part et d'autre de la position centrale de l'urètre.

Les aiguilles implantées successivement à l'aide de la tige de manipulation, remontent ensuite pour contourner la vessie et l'os pubien, et ressortent du corps par des incisions effectuées dans la paroi abdominale en région sus-pubienne.

Les deux moitiés de gaine qui se chevauchent en regard du milieu de la bandelette sont retirées en tirant sur les extrémités sortant des incisions sus-pubiennes.

Un tel dispositif nécessite une utilisation d'ancillaires du type poignée d'introduction et guide rigide de cathéter intravésical, spécialement conçus pour ce type d'intervention chirurgicale.

L'intervention chirurgicale connue présente également un inconvénient dans la mesure où les aiguilles sont introduites dans la paroi antérieure du vagin pour ressortir en région sus-pubienne. Ce chemin de bas en haut n'est pas contrôlable de façon précise pour contourner la base de la vessie. Des perforations vésicales sont loin d'être rares. Elles doivent être reconnues en peropératoire par deux cystoscopies et obligent à répéter les manoeuvres dans des conditions plus difficiles.

Les deux accessoires (poignée de manipulation et guide sonde rigide) doivent donc être à la disposition de l'équipe chirurgicale lors de chaque intervention, en étant préalablement lavés, emballés et stérilisés avant chaque utilisation. De plus ce passage latéral par rapport à la vessie, avec un point d'entrée vaginal risque d'offenser avec la pointe de l'aiguille, les vaisseaux iliaques dans la région rétrocrurale. Ces lésions vasculaires ont déjà été observées et ont pu entraîner des décès.

Un autre inconvénient du dispositif connu réside dans la difficulté de répéter l'intervention, avec le même dispositif, lorsque l'on constate par cystoscopie que l'ensemble gaine-bandelette a emprunté un mauvais trajet. Le retour en arrière peut s'avérer difficile et délicat pour la gaine et surtout pour les volumineuses aiguilles.

L'ensemble gaine-bandelette doit alors être sectionné et le dispositif n'est plus utilisable pour emprunter un autre trajet, il risque également de ne plus être stérile en étant contaminé lors de ces manoeuvres complémentaires.

L'objet de la présente invention vise à remédier aux inconvénients de l'état de la technique de manière à obtenir une technique opératoire différente, plus facile, plus rapide et plus sûre (par rapport à la vessie et aux vaisseaux). Le dispositif proposé permet de mettre en oeuvre cette technique différente qui sera décrite en détails plus loin dans la présente description.

Un autre objet de la présente invention vise à réaliser un dispositif pour le traitement de l'incontinence urinaire susceptible d'être réutilisé facilement en cas d'introduction dans le corps selon un trajet non optimal, éventualité qui selon la présente invention ne peut être qu'exceptionnelle en cas de très fortes adhérences de la vessie au pubis à la suite d'interventions antérieures.

Selon l'invention, l'extrémité proximale de l'aiguille de perforation est reliée à la première extrémité du moyen souple grâce à un élément de traction intermédiaire, la seconde extrémité du moyen souple étant libre ou prolongée par un élément de traction intermédiaire complémentaire.

Selon un mode de réalisation du descriptif conforme à l'invention, la gaine de protection enveloppe complètement la bandelette y compris ses premières et seconde extrémités.

Selon un mode de réalisation de l'invention, la gaine est sécable en deux parties séparables par glissement dans deux sens opposés par rapport à la bandelette, ledit dispositif comportant un moyen sécable entre les deux extrémités centrales et adjacentes de la gaine.

Selon un autre mode de réalisation du dispositif conforme à l'invention, il comporte un fil agencé sensiblement perpendiculaire à l'axe longitudinal de la gaine, de sorte à découper ladite gaine lorsqu'une traction est exercée sur ledit fil.

Selon un mode de réalisation du dispositif conforme à l'invention, la gaine est réalisée dans un matériau thermorétractable à base de fluoropolymère.

Selon un mode de réalisation du dispositif conforme à l'invention, la bandelette est formée à partir d'un matériau tricoté macro poreux.
Selon un mode de réalisation de l'invention, la bandelette présente dans sa partie centrale un film hydrophile résorbable diminuant le risque d'adhérence ou le risque d'érosion de l'urètre.

Selon un mode de réalisation du dispositif conforme à l'invention, l'aiguille perforante présente une partie courbe continûment adjacente avec une partie sensiblement droite se terminant par son extrémité proximale.

Selon un mode de réalisation de l'invention, l'élément de traction intermédiaire est un lacet de traction.

Selon un mode de réalisation, le dispositif conforme à l'invention comporte un embout sur lequel la gaine est accrochée par thermorétraction et sur lequel vient s'accrocher le lacet de traction.

Selon un mode de réalisation conforme à l'invention, le lacet de traction présente une longueur sensiblement égale à la longueur du moyen souple.

Selon un mode préféré de réalisation du dispositif conforme à l'invention, l'élément de traction intermédiaire est tubulaire.

Selon un mode de réalisation du dispositif conforme à l'invention, l'élément de traction intermédiaire et l'aiguille de perforation sont assemblés par vissage.

Selon un mode de réalisation du dispositif conforme à l'invention, l'élément de traction est constitué de deux parties substantiellement de même longueur, raboutées et assemblées l'une à l'autre de façon amovible, par exemple par vissage à l'aide d'un raccord.

Selon un mode de réalisation du dispositif conforme à l'invention, l'aiguille de perforation est unique.

D'autres caractéristiques et avantages ressortiront également de la description détaillée, non limitative, donnée ci-après en référence au dessin annexé dans lequel :
- la figure 1 représente une vue en coupe du moyen souple du dispositif conforme à l'invention,
- la figure 2 représente une vue de dessus, avec arrachement partiel du moyen souple montré à la figure 1.
- la figure 3 représente partiellement un autre exemple de réalisation du moyen souple de la figure 2,
- la figure 4 représente une vue partielle d'un dispositif conforme à l'invention,
- les figures 5 et 6 représentent des détails de la figure 4,
- les figures 7 et 8 représentent un autre mode de réalisation du moyen souple du dispositif conforme à l'invention,
- la figure 9 représente une vue partielle d'un autre mode de réalisation du dispositif conforme à l'invention,
- les figures 10, 11a, 11b et 11c représentent des détails du dispositif représenté à la figure 9,
- les figures 12 à 19 représentent schématiquement la méthode chirurgicale mise en oeuvre à l'aide du dispositif conforme à l'invention,
- les figures 20 et 21 représentent schématiquement la position du dispositif conforme à l'invention dans le corps d'une patiente.

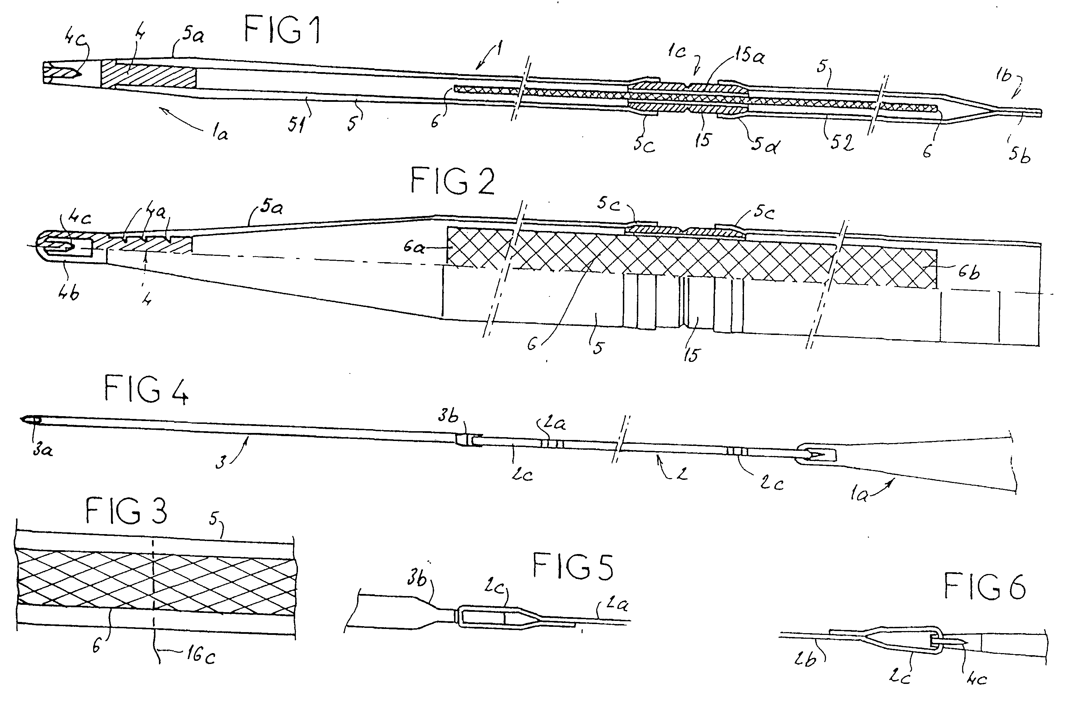

Le dispositif représenté partiellement aux figures 1, 2, 3 et 4 comprend un moyen souple 1 allongé constituant généralement en une bande composite.

Le moyen souple 1 n'est montré qu'en partie et non dans toute sa longueur. Il présente généralement une forme aplatie et allongée.

La première extrémité 1a du moyen souple 1 est susceptible d'être liée à un élément de traction intermédiaire 2 (cf Figure 4) et la seconde extrémité 1b est libre ou prolongée par un élément de traction intermédiaire complémentaire (non représenté).

Le dispositif conforme à l'invention comporte également une aiguille de perforation 3, par exemple en inox, liée à l'élément de traction intermédiaire.

Le moyen souple 1 comprend une gaine de protection 5, enveloppant à plat une bandelette 6.

L'aiguille de perforation 3 unique présente une extrémité distale 3a active et une extrémité proximale 3b, reliée à la première extrémité 1a du moyen souple 1.

L'extrémité proximale 3b de l'aiguille de perforation 3 est reliée à la première extrémité 1a, grâce à l'élément de traction intermédiaire 2. L'extrémité proximale 3b est ainsi une extrémité non perforante.

L'extrémité distale 3a est au bout d'une partie courbe continûment adjacente avec une partie sensiblement droite se terminant par l'extrémité proximale 3b.

L'aiguille de perforation présente par exemple un diamètre de 3,5 mm dans sa partie courbe.

L'extrémité proximale 3b est également pourvue d'un moyen d'accrochage permettant de réaliser la liaison avec l'élément de traction intermédiaire.

La gaine 5 de protection enveloppe complètement la bandelette 6, y compris sa première extrémité 6a et sa seconde extrémité 6b .

La gaine 5 est par exemple réalisée avec un matériau thermorétractable à base de fluoropolymère. Le matériau constitutif de la gaine 5 est choisi de sorte qu'il soit parfaitement étanche pour isoler complètement la bandelette 6 destinée à être implantée dans le corps de la patiente, des contacts cutanés et muqueux lors de la réalisation des trajets d'implantation dans ledit corps.

La gaine 5 peut également présenter des propriétés de faible coefficient de frottement. Ces propriétés se retrouvent alors à l'intérieur et à l'extérieur de la gaine 5, de manière à assurer, d'une part une bonne séparation par rapport à la bandelette 6, et d'autre part une réduction du frottement à l'intérieur du corps de la patiente lorsque le moyen souple 1 est tracté.

La bandelette 6 présente avantageusement une largeur comprise entre 6 et 14 mm, préférentiellement entre 10 et 12 mm et une longueur comprise entre 30 et 50 cm, de préférence autour de 40 cm.

La bandelette 6 est de préférence formée à partir d'un matériau tricoté macro poreux.

Ce dernier est constitué par exemple d'un tricot ajouré en monofil de polypropylène ayant une grosseur comprise entre 0,12 et 0,16 millimètre et composé de deux nappes formées par deux barres à passettes enfilées chacune, une passette pleine-une passette vide, ces deux barres étant déplacées symétriquement en mailles ouvertes suivant le barème suivant :
- barre I : 01-12-32
- barre II : 32-21-01

La bandelette 6 est découpée en longueur dans le sens de la chaîne du tricot.

Ce dernier, par exemple d'une largeur de 12mm présente donc les caractéristiques suivantes :
- une résistance à la rupture dans le sens de la chaîne de 105N ± 20%,
- un allongement à la rupture dans le sens de la chaîne de 92% ± 20%
- un allongement sous une force de 20N de 36%
- un début de tuilage avec une face de 6N et un allongement de 15%
Par "tuilage", on entend la propriété selon laquelle la bandelette 6 s'enroule spontanément par rapport à elle-même, autour de son axe longitudinal, sous contrainte en tension longitudinale.

La bandelette 6 présente des avantages intéressants et en particulier une faible émission de particules lors de son étirement, ainsi qu'un tuilage qui n'apparaît que sous une contrainte importante (6N). Toutes ces caractéristiques précitées n'altèrent en rien la porosité de la bandelette 6.

La gaine 5 est de préférence sécable en deux parties 51, 52, séparables par glissement dans deux sens opposés par rapport à la bandelette 6.

Le dispositif conforme à l'invention comporte à cet effet un moyen sécable 15, sensiblement au centre de la gaine 5, et dont les deux extrémités sont solidarisées avec les extrémités centrales 5c, 5d correspondantes et adjacentes de ladite gaine 5.

Le matériau du moyen sécable 15 est choisi parmi les matériaux du type thermoplastique agréés pour application chirurgicales.

La liaison entre, d'une part, les extrémités centrales 5c, 5d des deux parties 51 et 52 respectivement, et, d'autre part, le noyau sécable 15, est obtenue par tout moyen susceptible de rendre ladite gaine 5 étanche. Il en est de même pour l'extrémité libre 5b de la gaine 5, laquelle est tout simplement obturée ou scellée.

Le moyen sécable 15 compote une fente plate 15a le traversant d'une extrémité longitudinale à l'autre, pour le passage libre de la bandelette 6.

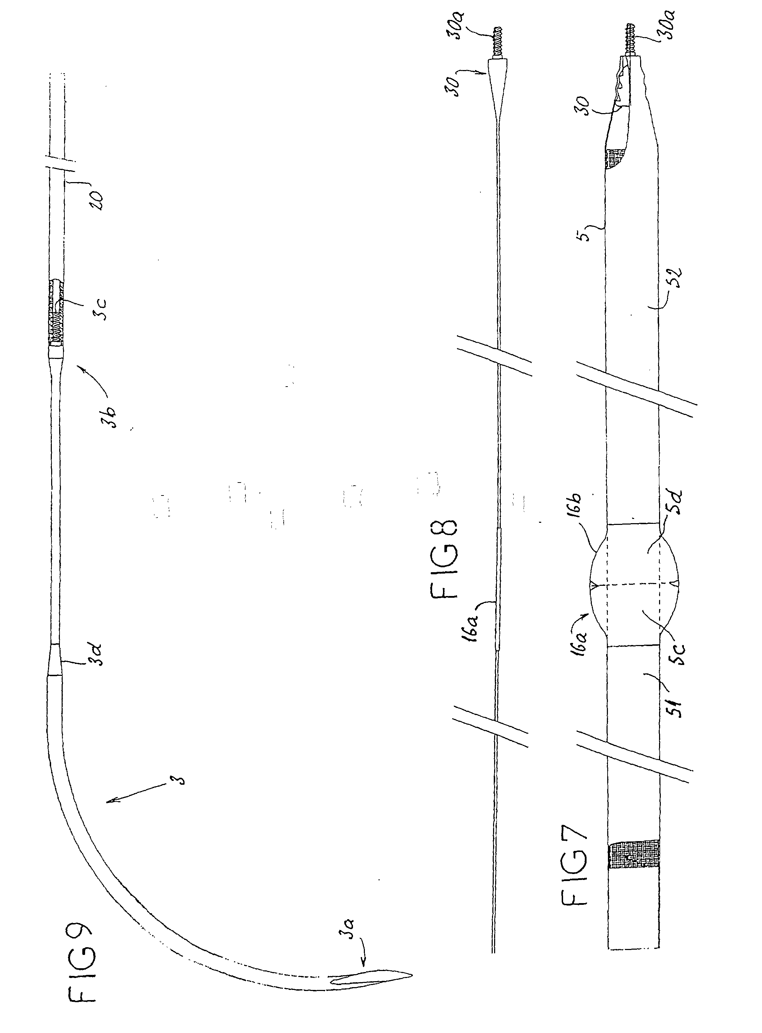

Le moyen sécable 15 peut être remplacé dans un mode de réalisation représenté aux figures 7 et 8, par un manchon adhésif 16a raboutant les deux parties 51 et 52 sécables de la gaine 5. Ce manchon est fragilisé par exemple par une découpe partielle en pointillés ou une ligne d'affaiblissement de la gaine 5. Le manchon adhésif 16a réalisé avec un matériau souple adhérant intimement aux extrémités centrales 5c, 5d, présente également une languette prédécoupée 16b pour faciliter la déchirure dudit manchon 16a, et par conséquent, la séparation de la gaine 5 en les deux parties 51 et 52.

Selon un autre mode de réalisation du dispositif conforme à l'invention, la gaine 5 monobloc incorpore un fil 16c agencé sensiblement perpendiculairement à l'axe longitudinal de la gaine 5, de sorte à découper ladite gaine 5 lorsqu'une traction est exercée sur ledit fil 16c. Un tel exemple est par exemple représenté à la figure 3. Le fil 16c est avantageusement en couleur de manière à le repérer facilement.

La gaine 5 est ainsi sectionnable dans la zone centrale 1 c du moyen souple 1, de manière à libérer la bandelette 6 à l'intérieur du corps de la patiente.

L'élément intermédiaire de traction est, selon un mode d'exécution de l'invention, par exemple réalisé avec un lacet de traction 2 représenté à la figure 4.

Le dispositif conforme à l'invention comporte également un embout 4 sur lequel la gaine 5 est accrochée par thermo-rétraction. La gaine 5, plus particulièrement sa première extrémité 5a, est ainsi thermo-rétractée de manière étanche sur l'embout 4.

Comme représenté à la figure 2, l'embout 4 comporte des encoches 4a d'ancrage dans lesquelles s'engage la matière thermo-rétractée constitutive de la gaine 5. L'embout 4 est réalisé par exemple avec une pièce en inox ou en toute autre matière rigide, susceptible de venir en contact avec des tissus intracorporels.

Avantageusement, le moyen souple 1 comporte un oeillet de nouage 4b (cf. figure 4), disposé à l'extérieur de la gaine 5. Cet oeillet 4b est par exemple constitué d'une boucle fermée et peut comporter le cas échéant une pointe 4c, destinée à traverser et à accrocher une extrémité du lacet de traction 2 ou tout autre élément intermédiaire de traction.

L'oeillet de nouage 4b est par exemple réalisé de manière monobloc dans l'embout 4. Ce dernier peut également présenter une forme sensiblement méplate et partiellement tronconique de manière à réaliser une continuité entre des épaisseurs différentes du lacet de traction 2 et du moyen souple 1. Ce dernier présente une largeur plus importante que la largeur du lacet de traction 2. On obtient ainsi une certaine continuité entre les dimensions du lacet de traction 2 et du moyen souple 1. Le lacet de traction peut par exemple être réalisé avec un matériau téfloné.

La figure 4 représente schématiquement un exemple de réalisation du dispositif conforme à l'invention.

L'aiguille 3 comprend l'extrémité proximale 3b, sur laquelle est accrochée la première extrémité 2a du lacet de traction 2. Ce dernier présente par exemple à chacune de ses extrémités 2a et 2b, une boucle 2c (cf respectivement figures 5 et 6), obtenue par soudure à ultrasons, couture ou par tout autre moyen.

L'une de ces boucles 2c est montée sur l'extrémité proximale 3b de l'aiguille 3, alors que l'autre est montée dans l'oeillet 4b.

Lors du mode opératoire, il est ainsi possible d'accrocher l'extrémité 2a du lacet de traction 2 sur l'oeillet 4b, soit à l'aide de la pointe 4c solidaire dudit oeillet 4b, ou tout simplement en effectuant un point au fil sur la boucle fermée constitutive de l'oeillet 4b.

Le dispositif conforme à l'invention présente donc par exemple un lacet de traction 2, dont la longueur est par exemple sensiblement égale à la longueur du moyen souple 1, soit une longueur comprise entre 30 et 60 cm. De telles dimensions ou longueurs du lacet de traction 2 permettent, si l'on se réfère au mode opératoire, d'éviter d'engager le moyen souple 1 dans le corps avant de s'être assuré que le trajet effectué par l'aiguille 3 et le lacet de traction 2 est optimal. La grande longueur disponible du lacet de traction 2 pour effectuer les gestes opératoires décrits, permet ainsi de refaire en cas de besoin, un autre trajet dans le corps, en évitant de manipuler le moyen souple 1.

A titre de variante, il est possible de pourvoir la seconde extrémité 1b du moyen souple 1, d'un élément intermédiaire de traction complémentaire, tel qu'un lacet complémentaire conforme à celui précédemment décrit sous la référence 2.

Un lacet de traction 2 complémentaire et solidaire de l'extrémité libre 1b, laquelle peut également comporter un embout 4 à cet effet, permet d'effectuer une traction en sens opposé à son introduction sur le moyen souple 1, et de le faire ressortir de son trajet, en cas de mauvaise pénétration.

Selon un autre mode de réalisation du dispositif conforme à l'invention, représenté par exemple à la figure 9, l'élément intermédiaire de traction est un élément tubulaire 20. Le matériau constitutif de ce dernier est par exemple du PVC.

L'élément tubulaire 20 est de préférence semi-rigide, de manière à pouvoir être vissé sur une extrémité filetée 3c, solidaire de l'extrémité proximale 3b de l'aiguille 3. L'autre extrémité de l'élément tubulaire 20 est raccordée, par exemple par vissage, sur le moyen souple 1, comportant un embout 30 solidaire de la gaine 5, par exemple par thermo-rétraction. L'embout 30 présente à cet effet une partie complémentaire filetée 30a (voir figures 7 et 8).

Le moyen souple 1, l'élément tubulaire 20 et les deux parties 3a, 3b de l'aiguille de perforation 3 peuvent ainsi être assemblés de façon amovible par vissage.

D'autres moyens connus de connexion ou d'accrochage amovibles peuvent également convenir dans le cadre de la présente invention.

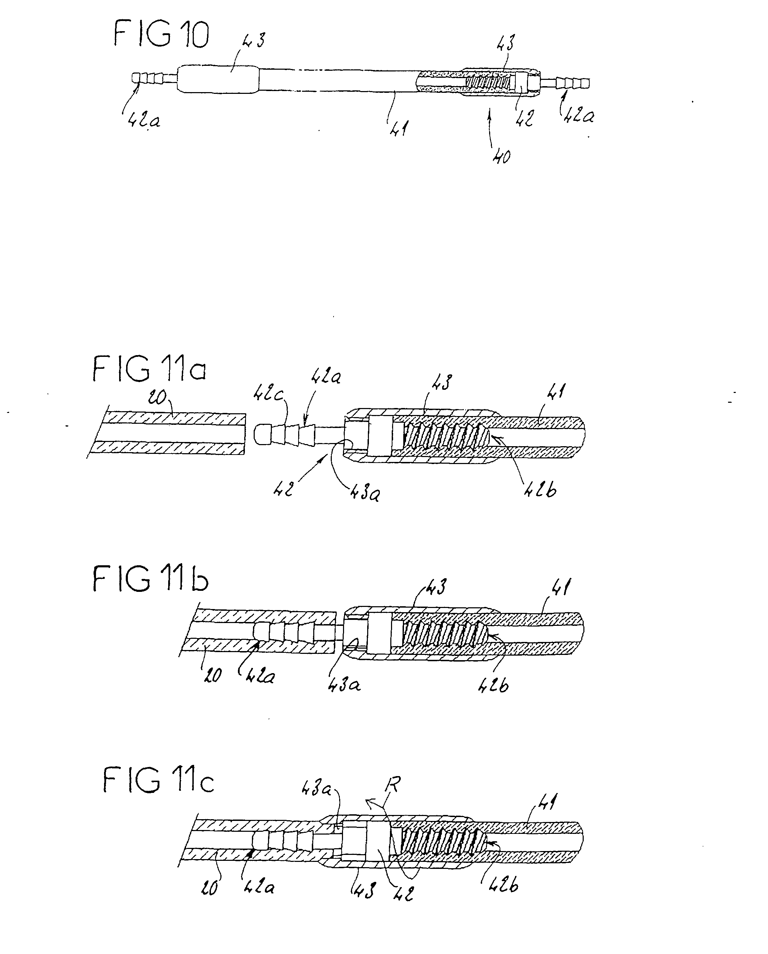

L'élément tubulaire est par exemple constitué de plusieurs parties substantiellement de même longueur par exemple 20 et 41, raboutées et assemblées, l'une à l'autre de façon amovible par vissage. Cet raboutage est obtenu à l'aide d'un raccord 40 représenté aux figures 10, 11a, 11b et 11c.

Le raccord 40 est composé de deux éléments, à savoir un mandrin interne de connexion 42 et une douille externe 43 de serrage.

Le mandrin 42 présente d'un côté une tétine filetée 42b engagée dans la partie d'élément tubulaire 41, et de l'autre côté un têton 42a pourvu de crans 42c, destiné à être engagée dans la partie d'élément tubulaire 20, tel que représenté aux figures 11 b et 11c. La forme et les dimensions des crans 42c ont tendance à s'opposer à la séparation de la partie d'élément tubulaire 20 du mandrin 42.

Chaque mandrin 42 est associé à une douille 43 engagée sur la partie d'élément tubulaire 41, de manière à enserrer une partie de ladite partie 41 entre la tétine filetée 42b et ladite douille 43.

Cette dernière est également pourvue du côté antérieur d'un taraudage 43a, qui vient s'encastrer par vissage dans la matière constitutive de l'extrémité raboutée de la partie 20 d'.

Préalablement une extrémité de la partie 41 d'élément tubulaire est visée sur la tétine 42b ; puis la douille 43 est rapportée sur cette extrémité de la partie 41, avec encastrement de la matière constitutive entre le filetage de la tétine 42b et le manchon 43, ce dernier enserrant également le reste du mandrin 42 sauf le téton 42a saillant.

L'assemblage des deux parties 20 et 41 d'élément tubulaire est effectué en engageant le téton 42a dans une extrémité de ladite partie 20 (figure 11b), puis en vissant l'extrémité de la douille 43 sur la périphérie extérieure de ladite partie 20 (figure 11c). La rotation de la douille 43 est schématisée par la flèche R sur la figure 11c. La liaison mécanique entre la partie d'élément tubulaire 20 et le raccord 40 est ainsi améliorée.

Le dispositif conforme à l'invention permet de mettre en oeuvre un procédé de traitement chez la femme souffrant d'incontinence urinaire d'effort. Ce procédé sera précisé ci-après et permet de mettre en place la bandelette 6 dans le corps de la patiente.

Le procédé de traitement comprend les étapes consistant à :
a) former une ouverture 103a dans la paroi vaginale antérieure 103,
b) effectuer deux petites incisions sus-pubiennes 106, 107,
c) utiliser l'aiguille de perforation 3 reliée à un moyen intermédiaire de traction 2 ou 20 pour créer un premier trajet contournant l'os pubien 105 et débouchant dans l'ouverture 103a formée dans la paroi vaginale antérieure 103,
d) utiliser l'aiguille de perforation 3, reliée à un moyen intermédiaire de traction, pour créer le second trajet contournant l'os pubien 105 et débouchant dans l'ouverture 103a formée dans la paroi vaginale antérieure 103.
e) vérifier par cystoscopie que la réalisation de ces trajets n'a pas perforé la vessie 101 ou l'urètre 100,
f) relier les parties du dispositif débouchant de l'ouverture 103a formée dans la paroi vaginale antérieure 103,
g) tirer sur l'élément intermédiaire de traction 2 ou 20 pour ajuster la boucle formée par le moyen souple 1 sous la surface inférieure de l'urètre 100,
h) séparer et retirer les deux moitiés de la gaine 5.
i) et laisser la bandelette 6, entre la première 106 et la seconde incision sus-pubienne 107 en passant sous la surface inférieure de l'urètre 100.

Selon un mode d'utilisation de l'invention, le procédé consiste à guider les trajets de l'aiguille de perforation 3 le long de la surface postérieure 105a du pubis, grâce au contact avec le doigt 50 du chirurgien introduit à travers l'ouverture 103a, formée dans la paroi vaginale antérieure 103, jusqu'au bord inférieur 105bdu même côté du pubis 105.

Selon un mode d'utilisation du dispositif conforme à l'invention, le moyen souple 1 est accroché à l'élément intermédiaire 2 ou 20, après la vérification prévue à l'étape (e).

Selon un mode d'utilisation du dispositif conforme à l'invention, l'ouverture 103a ménagée dans la paroi vaginale antérieure 103 est verticale.

Selon le procédé, on insère à la patiente au préalable une sonde urétrale 60, à ballonnet du type FOLEY.

Selon un procédé de traitement conforme utilisant le dispositif à l'invention, on utilise deux éléments intermédiaires de traction 2 ou 20, raccordés bout à bout au cours de l'étape (f).

Selon un autre procédé de traitement utilisant le dispositif conforme à l'invention, on sépare l'aiguille de perforation 3 et l'élément intermédiaire de traction 2 ou 20, après avoir mis en oeuvre la vérification selon l'étape (e) et avoir introduit le moyen souple 1 selon le premier trajet. Cette séparation permet alors de créer avec l'aiguille de perforation 3 reliée à l'élément intermédiaire de traction 2 ou 20, le second trajet.

Toutes les étapes précédemment définies sont maintenant décrites avec un dispositif conforme aux figures 7 à 11 et à la description s'y rapportant.

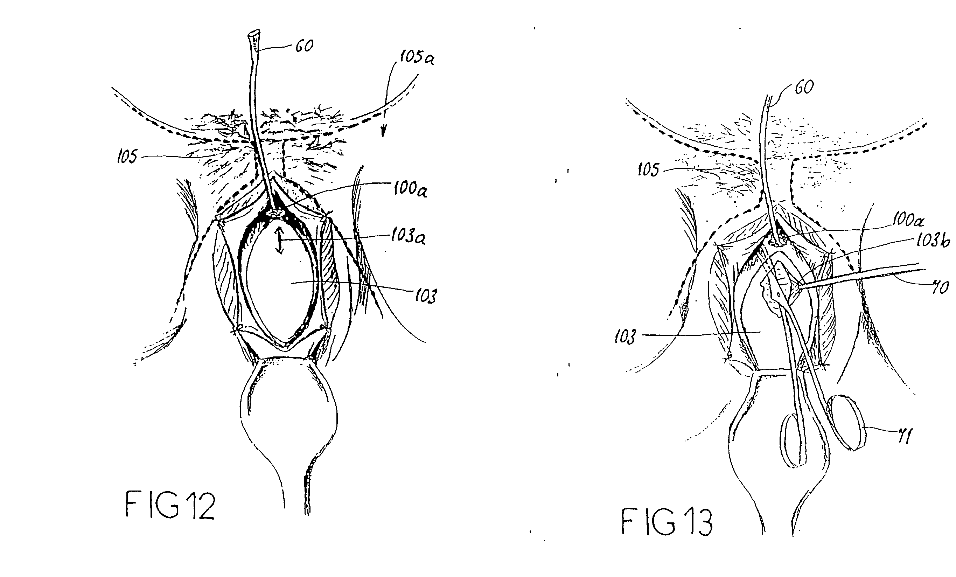

La première étape (a) est illustrée à la figure 12. La patiente est placée en position gynécologique et on constitue une zone d'opération stérile. Une sonde à ballonnet urétrale 60 est positionnée dans la vessie 101 et est connectée à une poche collectrice stérile pour vider et mettre à plat la vessie 101. Une courte incision verticale 103a, d'au maximum 30 mm de longueur, est pratiquée au milieu de la paroi vaginale 103, centrée dans le tiers central du canal urétral 100 débouchant sur le méat urinaire 100a. Chaque lèvre 103b de l'incision vaginale 103a est décollée des tissus sous-jacents à l'aide de ciseaux 71 et d'instruments 70 adéquats, comme illustré à la figure 13.

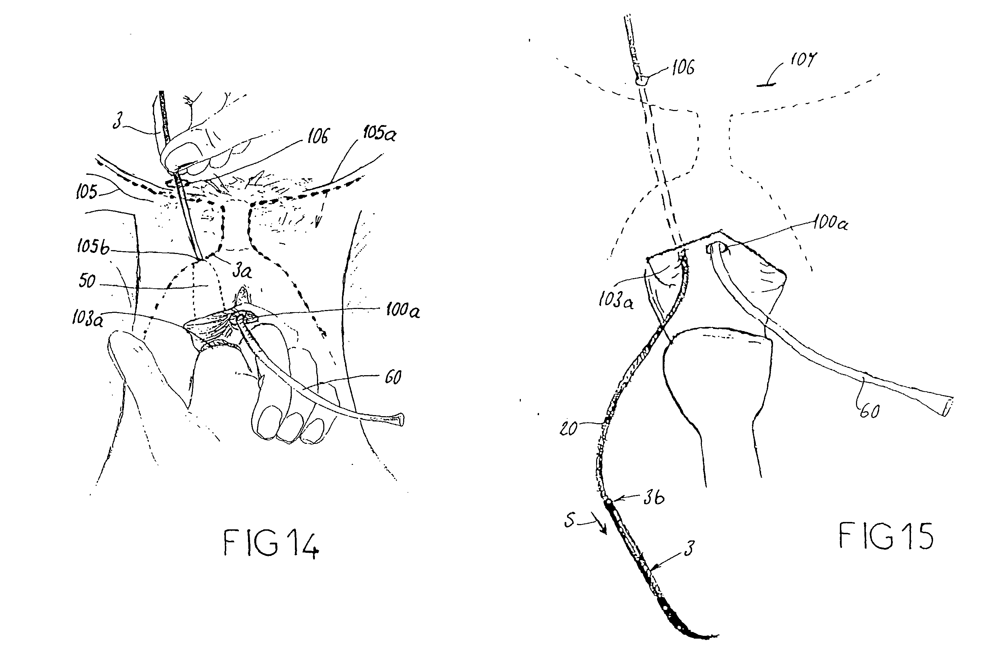

Le décollement est effectué jusqu'à ce que l'index 50 du chirurgien, introduit par l'ouverture 103a ainsi obtenue, puisse atteindre le bord inférieur 105b du pubis 105, en dehors de l'urètre 100 et des tissus périurétraux (figure 14).

Ensuite, une très petite incision cutanée 106, 107 de moins de 10 mm de long est pratiquée dans la peau abdominale immédiatement au-dessus du pubis 105, de chaque côté de la ligne médiane et à environ 20 mm de celle-ci, pour permettre le passage percutané de l'aiguille 3 en rasant la face postérieure 105a du pubis 30 dans la direction du vagin 104.

L'index 50 du chirurgien est introduit dans le passage vaginal ainsi préparé par décollement, et l'extrémité distale active 3a de l'aiguille 3 suit un trajet pour venir en contact direct avec cet index 50. Le trajet de l'aiguille de perforation 3 est donc parfaitement contrôlé. L'aiguille 3 peut ensuite ressortir par l'ouverture vaginale 103a et la vessie 101 est restée totalement à l'abri de toute offense de l'aiguille.

En général, le trajet percutané droit est réalisé en premier. L'extrémité de l'élément de traction intermédiaire, par exemple l'élément tubulaire 20, ressort derrière l'aiguille 3 par l'ouverture vaginale 103a. Cette extrémité de l'élément de traction 20 est par exemple dévissée de l'extrémité proximale 3b de l'aiguille 3, et séparé de cette dernière suivant la flèche S de la figure 15.

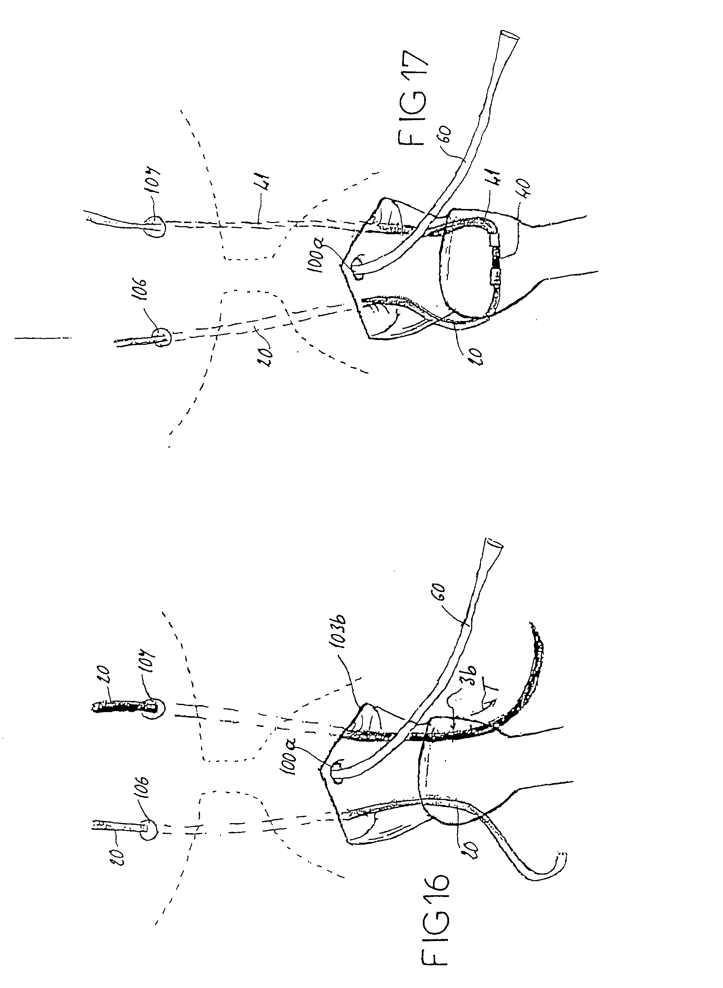

L'aiguille 3 est ensuite passée dans le deuxième trajet (côté gauche, figure 16), de la même manière que pour le premier côté (droit). Une fois que la partie distale 3a de l'aiguille 3 est bien en dehors de l'ouverture vaginale 103a, elle peut être détachée par dévissage de sa portion proximale 3b. Cette séparation est schématisée par la flèche T à la figure 16.

La portion dite proximale 3b est suffisamment longue pour faire saillie lors de l'incision percutanée 107.

A titre de variante conforme à l'invention, la portion proximale 3b peut être prolongée par un élément tubulaire 20 complémentaire, lequel fait saillie hors de l'incision abdominale 107.

L'étape suivante consiste à connecter l'extrémité de l'élément de traction tubulaire 20 empruntant le premier trajet à la portion proximale 3b ou à l'élément tubulaire 20 complémentaire empruntant le second trajet.

La connexion est effectuée à l'aide du raccord 40, représenté à la figure 17.

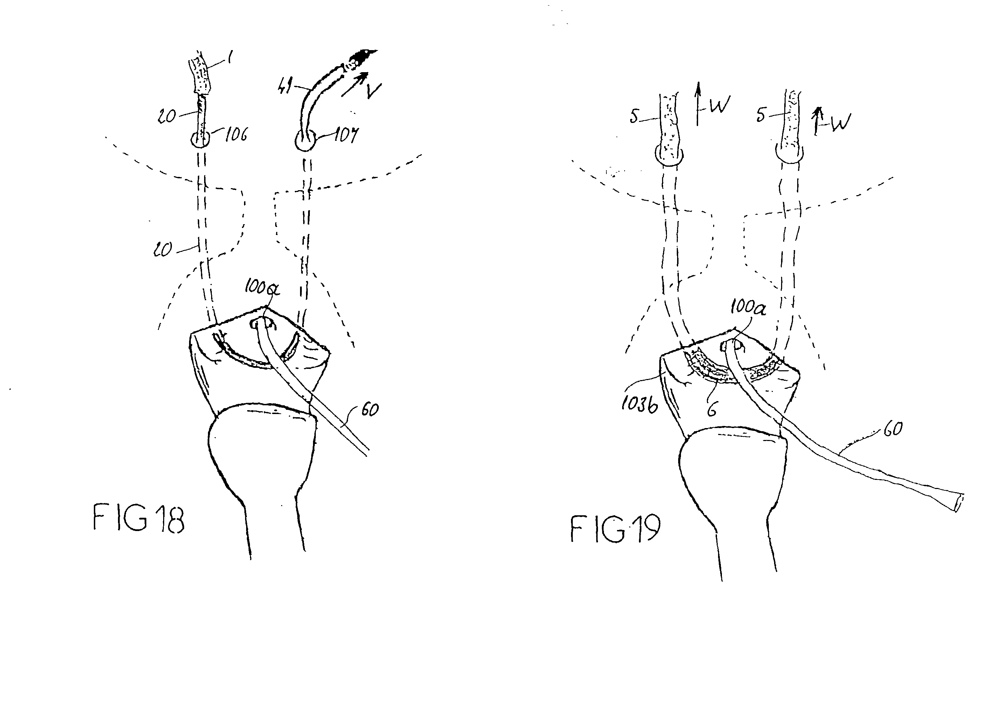

La portion proximale 3b est ensuite ressortie par l'abdomen en tirant selon la direction de la flèche V qui est illustrée dans la figure 18.

Avant que ne pénètre le moyen souple 1 dans le corps de la patiente, l'élément tubulaire 20 forme ainsi une boucle autour de l'urètre et ses deux extrémités ressortent respectivement par les deux incisions cutanées abdominales 106 et 107 (figure 18).

La sonde urétrale 60 est ensuite retirée et on effectue une cystoscopie pour vérifier l'absence de perforation vésicale.

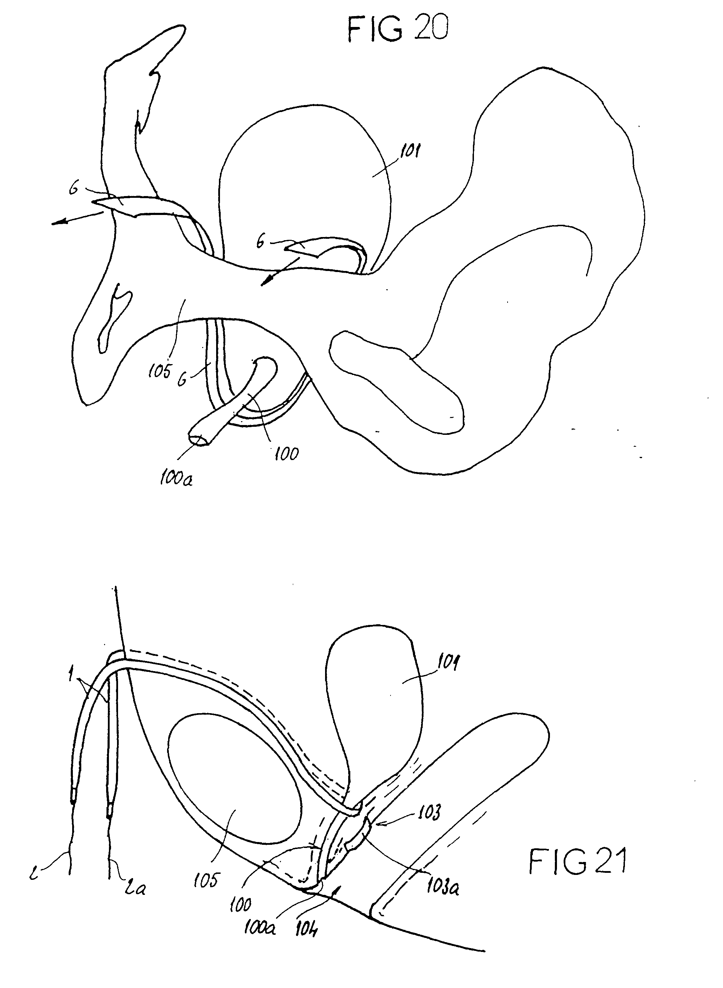

Une fois la vérification effectuée, l'extrémité de l'élément tubulaire 20 débouchant de l'incision abdominale 106 est assemblée par vissage sur l'extrémité filetée 30a du moyen souple 1 (gaine 5 plus bandelette 6), et l'ensemble est tiré (flèche V de la ligne 18) à travers les trajets droit et gauche pour positionner le moyen souple 1 sous l'urètre 100. Lorsque ce positionnement correspondant à l'étape (g) est terminé, les extrémités 1a, 1b du moyen souple 1 ressortent des incisions abdominales 106, 107, comme représenté à la figure 21.

On procède ensuite à la séparation de la gaine 5 de la bandelette 6, en sectionnant ladite gaine 5 dans sa zone centrale 1c et en retirant chacune des moitiés 51 et 52, ainsi obtenues, par l'incision abdominale 106, 107 correspondante, conformément à l'étape (h) (flèches W de la figure 19).

La bandelette 6 est ainsi libérée, positionnée et ajustée sous la face inférieure de l'urètre 100, habituellement dans une position centrale, sans tension et sans écrasement, comme représenté à la figure 20.

Une fois le retrait de la gaine 5 effectué à travers chaque incision abdominale 106 et 107, on sectionne au ras de la paroi abdominale, les parties de la bandelette 6 faisant saillie hors desdites incisions 106 et 107, lesdites parties étant laissées en site sous-cutané.

Les incisions cutanées sont refermées selon les méthodes conventionnelles.

La bandelette 6 contourne avantageusement l'os pubien 105 par sa face pelvienne ou profonde, pour se diriger vers la paroi abdominale controlatérale.

On procède ensuite à la fermeture de l'incision vaginale résultante 103a, effectuée dans la paroi vaginale 103, après avoir vérifié la position définitive de la bandelette 6 sous l'urètre 100.

A titre de variante, le lacet de traction 2 convient également pour mettre en oeuvre ladite méthode

Le mode opératoire ainsi utilisé est remarquable dans le sens qu'il s'agit d'une technique opératoire percutanée, puisque la dimension des incisions cutanées abdominales est minime, juste destinée au passage des aiguilles, et que les trajets effectués par l'aiguille 3 s'effectuent de haut en bas, c'est-à-dire en pénétrant par des incisions abdominales 106 et 107, pour sortir par l'ouverture vaginale 103a correspondante, préalablement déterminée et préparée.

Ceci présente un énorme avantage sur le plan de la sécurité vis-à-vis des risques de perforation de la vessie 101 d'une part, et des vaisseaux iliaques d'autre part.

Ceci est un avantage considérable par rapport à la technique opératoire connue. Un contrôle de trajet peut être effectué par cystoscopie.

Lorsque l'on s'apercoit que le trajet ne convient pas, il est possible de retirer l'élément de traction intermédiaire 2 ou 20, pour effectuer un second trajet à travers l'incision cutanée abdominale 106, et ce sans introduire le moyen souple 1 dans le corps.

L'ensemble des éléments du dispositif conforme à l'invention peut donc être réutilisé en cas de mauvaise manipulation, ou si l'on veut améliorer ou optimiser le trajet à l'intérieur du corps de la patiente. Ceci représente un avantage considérable vis-à-vis de l'état de la technique connue.

En outre, la technique chirurgicale décrite ne nécessite ni l'utilisation d'introducteur pour l'aiguille, ni de tige endovésicale pour écarter la vessie 101 et l'urètre 100 à chaque passage de l'aiguille 3 lors d'un trajet de haut en bas. Ceci constitue une simplification intéressante , dans la mesure où la technique chirurgicale présentée dans l'état de la technique, nécessite l'utilisation d'une tige endovésicale à deux reprises, soit après chaque passage de chacune des deux aiguilles effectuant un trajet de bas en haut.

Il est à noter que le dispositif conforme à l'invention est un dispositif percutané, ce qui est avantageux vis-à-vis du dispositif connu qui pénètre par la muqueuse vaginale, et sort par la peau dans sa région sus-pubienne abdominale.

Il est remarquable que l'extrême simplicité du dispositif conforme à l'invention contribue non seulement à réduire son prix de revient et à son nombre d'éléments constitutifs, mais également à augmenter la sécurité du patient et la qualité du résultat, lors de la mise en oeuvre de la technique opératoire décrite dans la présente invention.

Le contrôle de l'effet obtenu sur la continence et le réglage de la tension de la bandelette 6 ne sont pas justifiés pour deux raisons. En effet, la continence en position allongée n'est pas comparable à la position debout et l'efficacité de la bandelette s'explique, non pas par un effet de serrage (qui risque d'entraîner une sténose), mais par un effet de support de soutènement.

L'opérateur choisit en outre selon le cas clinique étudié la position de la bandelette 6 par rapport au conduit urétral 100.

Dans l'état de la technique, ce choix n'est pas possible, et les essais de continence sont réalisés après remplissage vésical et effort de toux pour régler la tension de la bandelette bien que la technique soit dénommée "tension free".

## Revendications

1. Dispositif pour le traitement de l'incontinence urinaire d'effort chez la femme, comprenant :
- un moyen souple (1) et allongé comportant une bandelette (6) de soutien de l'urètre (100) et une gaine (5) de protection enveloppant à plat ladite bandelette (6) ;
- une aiguille perforante (3) présentant une extrémité distale active (3a) et une extrémité proximale (3b) liée à une première extrémité (1a) du moyen souple (1),
**caractérisé en ce que** l'extrémité proximale (3b) de l'aiguille de perforation (3) est reliée à la première extrémité (1a) du moyen souple (1) grâce à un élément de traction intermédiaire (2, 20), la seconde extrémité (1b) du moyen souple (1) étant libre ou prolongée par un élément de traction intermédiaire complémentaire.

2. Dispositif selon la revendication 1, **caractérisé en ce que** la gaine (5) de protection enveloppe complètement la bandelette (6), y compris ses première et seconde extrémités.

3. Dispositif selon la revendication 1 ou 2, dont la gaine est sécable en deux parties séparables par glissement dans deux sens opposés par rapport à la bandelette (6), **caractérisé en ce qu'**il comporte un moyen sécable (15) entre les deux extrémités centrales (5c, 5d) et adjacentes de la gaine (5).

4. Dispositif selon la revendication 1 ou 2, dont la gaine est sécable en deux parties séparables par glissement dans deux sens opposés par rapport à la bandelette (6), **caractérisé en ce qu'**il comporte un fil (16c) agencé sensiblement perpendiculaire à l'axe longitudinal de la gaine (5), de sorte à découper ladite gaine (5) lorsqu'une traction est exercée sur ledit fil (16c).

5. Dispositif selon l'une quelconque des revendications 1 à 4, **caractérisé en ce que** la gaine (5) est réalisée dans un matériau thermorétractable à base de fluoropolymère.

6. Dispositif selon l'une quelconque des revendications 1 à 5, **caractérisé en ce que** la bandelette (6) est formée à partir d'un matériau tricoté macro poreux.

7. Dispositif selon l'une quelconque des revendications 1 à 6, **caractérisé en ce que** la bandelette (6) présente dans sa partie centrale un film hydrophile résorbable diminuant le risque d'adhérence ou le risque d'érosion de l'urètre (100).

8. Dispositif selon l'une quelconque des revendications 1 à 7, **caractérisé en ce que** l'aiguille perforante (3) présente une partie courbe continûment adjacente avec une partie sensiblement droite se terminant par son extrémité proximale (3b).

9. Dispositif selon l'une quelconque des revendications 1 à 8, **caractérisé en ce que** l'élément de traction intermédiaire est un lacet de traction (2).

10. Dispositif selon la revendication 9, **caractérisé en ce qu'**il comprend un embout (4) sur lequel la gaine (5) est accrochée par thermorétraction et sur lequel vient s'accrocher le lacet de traction (2).

11. Dispositif selon la revendication 9 ou 10, **caractérisé en ce que** le lacet de traction (2) présente une longueur sensiblement égale à la longueur du moyen souple (1).

12. Dispositif selon l'une quelconque des revendications 1 à 8, **caractérisé en ce que** l'élément de traction intermédiaire est un élément tubulaire (20).

13. Dispositif selon la revendication 12, **caractérisé en ce que** l'élément de traction tubulaire (20) et l'aiguille de perforation (3) sont assemblés par vissage.

14. Dispositif selon la revendication 12 ou 13, **caractérisé en ce que** l'élément de traction tubulaire est constitué de deux parties substantiellement de même longueur, raboutées et assemblées l'une à l'autre de façon amovible, par exemple par vissage à l'aide d'un raccord (40).

15. Dispositif selon l'une quelconque des revendications 1 à 14, **caractérisé en ce que** l'aiguille de perforation (3) est unique.

16. Dispositif selon l'une quelconque des revendications 1 à 15 **caractérisé en ce que** la bandelette (6) est découpée dans un tricot ajouré composé deux nappes formées par deux barres à passettes enfilées chacune, une passette pleine-une passette vide, ces deux barres étant déplacées symétriquement en mailles ouvertes .

## Patentansprüche

1. Vorrichtung zum Behandeln von Harnstressinkontinenz bei Frauen, mit:
- flexiblen länglichen Mitteln (1), die einen Streifen (6) zum Unterstützen der Harnröhre (100) und eine den Streifen (6) flach umhüllende Schutzhülle (5) aufweisen;
- einer Punktionsnadel (3) mit einem wirkenden distalen Ende (3a) und einem proximalen Ende (3b), das mit einem ersten Ende (1a) der flexiblen Mittel (1) verbunden ist,
**dadurch gekennzeichnet, dass** das proximale Ende (3b) der Punktionsnadel (3) mit dem ersten Ende (1a) der flexiblen Mittel (1) über ein Zug vermittelndes Element (2, 20) verbunden ist, wobei das zweite Ende (1b) der flexiblen Mittel (1) frei ist oder durch ein zusätzliches Zug vermittelndes Element verlängert ist.

2. Vorrichtung nach Anspruch 1, **dadurch gekennzeichnet, dass** die Schutzhülle (5) den Streifen (6) einschließlich seines ersten und zweiten Endes vollständig einhüllt.

3. Vorrichtung nach Anspruch 1 oder 2, wobei die Hülle in zwei Teile teilbar ist, die durch Verschieben derselben in zwei entgegengesetzte Richtungen relativ zu dem Streifen (6) getrennt werden können, **dadurch gekennzeichnet, dass** die Vorrichtung teilbare Mittel (15) zwischen den beiden mittleren benachbarten Enden (5c, 5d) der Schutzhülle (5) aufweist.

4. Vorrichtung nach Anspruch 1 oder 2, wobei die Hülle in zwei Teile geteilt werden kann, die durch Verschieben derselben in zwei entgegengesetzte Richtungen relativ zu dem Streifen (6) getrennt werden können, **dadurch gekennzeichnet, dass** die Vorrichtung einen Faden (16c) aufweist, der etwa rechtwinklig zur Längsachse der Hülle (5) angeordnet ist, derart, dass die Hülle (5) zertrennt wird, wenn auf den Faden (16c) ein Zug ausgeübt wird.

5. Vorrichtung nach einem der Ansprüche 1 bis 4, **dadurch gekennzeichnet, dass** die Hülle (5) aus einem Fluorpolymerbasierten wärmeschrumpfbaren Material gefertigt ist.

6. Vorrichtung nach einem der Ansprüche 1 bis 5, **dadurch gekennzeichnet, dass** der Streifen (6) aus einem makroporösen gewirkten Material gebildet ist.

7. Vorrichtung nach einem der Ansprüche 1 bis 6, **dadurch gekennzeichnet, dass** der Streifen (6) in seinem mittleren Bereich einen resorbierbaren hydrophilen Film aufweist, der die Gefahr einer Adhäsion an oder die Gefahr einer Erosion der Harnröhre (100) vermindert.

8. Vorrichtung nach einem der Ansprüche 1 bis 7, **dadurch gekennzeichnet, dass** die Punktionsnadel (3) einen kontinuierlich gekrümmten Bereich aufweist, der einem in ihr proximales Ende (3b) endenden etwa geraden Bereich benachbart ist.

9. Vorrichtung nach einem der Ansprüche 1 bis 8, **dadurch gekennzeichnet, dass** das Zug vermittelnde Element eine Zugschnur (2) ist.

10. Vorrichtung nach Anspruch 9, **dadurch gekennzeichnet, dass** die Vorrichtung ein Endstück (4) aufweist, auf das die Hülle (5) durch Wärmeschrumpfen befestigt ist und an dem die Zugschnur (2) befestigt ist.

11. Vorrichtung nach Anspruch 9 oder 10, **dadurch gekennzeichnet, dass** die Zugschnur (2) eine Länge aufweist, die etwa gleich der Länge der flexiblen Mittel (1) ist.

12. Vorrichtung nach einem der Ansprüche 1 bis 8, **dadurch gekennzeichnet, dass** das Zug vermittelnde Element ein rohrförmiges Element (20) ist.

13. Vorrichtung nach Anspruch 12, **dadurch gekennzeichnet, dass** das rohrförmige Zugelement (20) und die Punktionsnadel (3) durch Verschraubung zusammengefügt sind.

14. Vorrichtung nach Anspruch 12 oder 13, **dadurch gekennzeichnet, dass** das rohrförmige Zugelement (20) aus zwei Teilen im wesentlichen gleicher Länge gebildet ist, die Ende an Ende angeordnet und beispielsweise durch Verschraubung unter Verwendung eines Verbindungsstückes (40) lösbar miteinander verbunden sind.

15. Vorrichtung nach einem der Ansprüche 1 bis 14, **dadurch gekennzeichnet, dass** die Punktionsnadel (3) die einzige Punktionsnadel ist.

16. Vorrichtung nach einem der Ansprüche 1 bis 15, **dadurch gekennzeichnet, dass** der Streifen (6) aus einem durchbrochenen Gewirk zugeschnitten ist, das aus zwei Lagen aufgebaut ist, die durch zwei Fadenführschienen - einen vollen Führer und einen leeren Führer - gebildet sind, wobei die beiden Schienen symmetrisch zu offenen Maschen bewegt werden.

## Claims

1. Device for treating urinary stress incontinence in women, comprising:
- a flexible and elongate means (1) comprising a tape (6) for supporting the urethra (100) and a protective sheath (5) lying flat and enveloping said tape (6);
- a puncturing needle (3) with an active distal end (3a) and a proximal end (3b) connected to a first end (1a) of the flexible means (1),
**characterized in that** the proximal end (3b) of the puncturing needle (3) is connected to the first end (1a) of the flexible means (1) by virtue of an intermediate traction element (2, 20), the second end (1b) of the flexible means (1) being free or extended by an additional intermediate traction element.

2. Device according to Claim 1, **characterized in that** the protective sheath (5) completely envelopes the tape (6), including its first and second ends.

3. Device according to Claim 1 or 2, the sheath of which can be split into two parts that can be separated by sliding them in two opposite directions relative to the tape (6), **characterized in that** it comprises a splittable means (15) between the two central and adjacent ends (5c, 5d) of the sheath (5).

4. Device according to Claim 1 or 2, the sheath of which can be split into two parts that can be separated by sliding them in two opposite directions relative to the tape (6), **characterized in that** it comprises a filament (16c) arranged roughly at right angles to the longitudinal axis of the sheath (5), so as to cut said sheath (5) when traction is exerted on said filament (16c).

5. Device according to any of Claims 1 to 4, **characterized in that** the sheath (5) is made of a fluoropolymer-based heat-shrinkable material.

6. Device according to any of Claims 1 to 5, **characterized in that** the tape (6) is formed from a macroporous knitted material.

7. Device according to any of Claims 1 to 6, **characterized in that** the tape (6) in its central region has a resorbable hydrophilic film reducing the risk of adhesion to or the risk of erosion of the urethra (100).

8. Device according to any of Claims 1 to 7, **characterized in that** the puncturing needle (3) has a curved part continuously adjacent to a roughly straight part ending in its proximal end (3b).

9. Device according to any of Claims 1 to 8, **characterized in that** the intermediate traction element is a traction lace (2).

10. Device according to Claim 9, **characterized in that** it comprises an end piece (4) onto which the sheath (5) is heat shrunk and to which the traction lace (2) attaches.

11. Device according to Claim 9 or 10, **characterized in that** the traction lace (2) has a length roughly equal to the length of the flexible means (1).

12. Device according to any of Claims 1 to 8, **characterized in that** the intermediate traction device is a tubular element (20).

13. Device according to Claim 12, **characterized in that** the tubular traction element (20) and the puncturing needle (3) are assembled by screwing.

14. Device according to Claim 12 or 13, **characterized in that** the tubular traction element consists of two parts of roughly the same length, placed end to end and joined together removably, for example by screwing using a coupling (40).

15. Device according to any of Claims 1 to 14, **characterized in that** the puncturing needle (3) is the only puncturing needle.

16. Device according to any of Claims 1 to 15, **characterized in that** the tape (6) is cut from an open knit made up of two layers formed by two threaded guide bars each - one full guide and one empty guide - these two bars being moved symmetrically for open mesh.
